(19) Europäisches Patentamt
European Patent Office
Office européen des brevets

(11) EP 1 202 982 B1

(12) FASCICULE DE BREVET EUROPEEN

(45) Date de publication et mention de la délivrance du brevet: **26.11.2003 Bulletin 2003/48**

(21) Numéro de dépôt: **00940693.5**

(22) Date de dépôt: **14.07.2000**

(51) Int Cl.[7]: **C07D 307/77**, C07D 307/83, A61K 7/06, A61K 7/16, A23G 3/30, A23L 1/226

(86) Numéro de dépôt international: **PCT/IB00/00957**

(87) Numéro de publication internationale: **WO 01/007428 (01.02.2001 Gazette 2001/05)**

(54) **PREPARATION D'UNE LACTONE BETA,GAMMA-INSATUREE ET SON UTILISATION A TITRE D'INGREDIENT AROMATISANT ET PARFUMANT**

HERSTELLUNG EINES GAMMA-UNGESÄTTIGTEN BETA-LACTONS UND SEINE VERWENDUNG ALS AROMATISIERENDER UND PARFÜMISIERENDER BESTANDTEIL

METHOD FOR PREPARING A GAMMA-UNSATURATED BETA-LACTONE AND USE THEREOF AS AN AROMATIC AND FLAVOURING INGREDIENT

(84) Etats contractants désignés:
**AT BE CH CY DE DK ES FI FR GB GR IE IT LI LU MC NL PT SE**

(30) Priorité: **22.07.1999 CH 134299**

(43) Date de publication de la demande: **08.05.2002 Bulletin 2002/19**

(73) Titulaire: **FIRMENICH SA
1211 Genève 8 (CH)**

(72) Inventeurs:
- **FREROT, Eric
F-74100 Ville la Grand (FR)**
- **BAGNOUD, Alain
CH-1213 Onex (CH)**

(74) Mandataire:
**Salvaterra-Garcia, Maria de Lurdes, Dr. et al
Firmenich SA,
1, route des Jeunes
1211 Genève 8 (CH)**

(56) Documents cités:
**EP-A- 0 041 122** **EP-A- 0 953 294**

- **C.S. FOOTE ET AL.: "Structure and Stereochemistry of the Ketoacids Derived from Menthofuran Photoperoxide" TETRAHEDRON, (INCL TETRAHEDRON REPORTS), vol. 23, 1967, pages 2601-2608, XP002155155 OXFORD GB**
- **J.A. HIRSCH ET AL. : "The Hydrolysis of alpha,alpha'-Dimethoxydihydromenthofuran" JOURNAL OF ORGANIC CHEMISTRY, vol. 32, no. 9, 1967, pages 2915-2916, XP002155156 EASTON US cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

### Domaine technique

La présente invention a trait au domaine de la parfumerie et de l'industrie des arômes. Elle concerne plus particulièrement l'utilisation de la (6R)-4,5,6,7-tétrahydro-3,6-diméthyl-3H-benzo[b]furan-2-one de formule

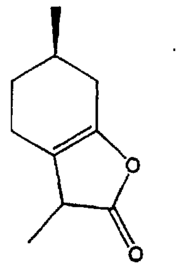

(I)

à titre d'ingrédient aromatisant ou parfumant. La lactone de formule (I) possède en effet d'excellentes qualités organoleptiques qui rendent son emploi très intéressant dans la parfumerie et dans l'industrie des arômes.

La présente invention concerne également un procédé original de préparation de la lactone de formule (I).

### Technique antérieure

La structure chimique de la (6R)-4,5,6,7-tétrahydro-3,6-diméthyl-3H-benzo[b]furan-2-one est connue. En effet, K. Schulte-Elte et al. dans Tetrahedron (1967), 23(6), 2583-2599, ont identifié le composé de formule (I) dans un mélange de lactones résultant de la photooxydation du menthofurane. Par ailleurs, J. Hirsch et al. dans J. Org. Chem. (1967), 32(9), 2915-2916, ont également identifié la lactone (I) parmi d'autres produits finaux résultant de l'hydrolyse du $\alpha,\alpha'$-diméthoxydihydromenthofurane. Cependant, ces auteurs précisent que la lactone (I) s'oxyde très facilement dans les conditions de la synthèse décrite pour donner une hydroxylactone, la 5,6,7,7a-tétrahydro-7a-hydroxy-3,6-diméthyl-4H-benzo[b]furan-2-one de formule

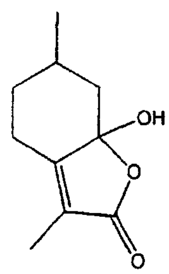

(II)

D'ailleurs certains auteurs tels que par exemple R. Woodward et al. dans J. Am. Chem. Soc. (1950), 72, 399-403, qui ont étudié l'oxydation du menthofurane en utilisant des conditions expérimentales très variées, n'ont jamais identifié la lactone (I), mais ont obtenu comme seul produit de l'oxydation l'hydroxylactone (II).

Ainsi, bien que la (6R)-4,5,6,7-tétrahydro-3,6-diméthyl-3H-benzo[b]furan-2-one ait été détectée parmi d'autres produits de réaction lors d'études de réactivité du menthofurane, aucun document de l'art antérieur ne décrit une synthèse optimale permettant d'obtenir la lactone (I), sans que celle-ci ne s'oxyde rapidement en hydroxylactone (II). En outre ces références ne donnent aucune indication ni même suggestion quant à une possible utilisation de la lactone (I) comme ingrédient parfumant ou aromatisant.

Par ailleurs, il est important de signaler que certains documents de l'art antérieur qui ont analysé les constituants de produits naturels tels que l'huile essentielle de *Pycnanthemum floridanum* (C. Shu et al., J. Ess. Oil Res. (1994), 6(5), 529-531) ou la menthe sauvage (K. Umemoto. Nagoya Gakuin Daigaku Ronshu. Jinbun, Shizen Kagakuhen (1983), 19(2), 79-86), ont identifié un produit qu'ils nomment "menthofurolactone", sans préciser sa structure. Or, il semble qu'il y ait une confusion dans la littérature quant à la structure associée à ce nom. En effet, alors que Chemical Abstract associe à la menthofurolactone la structure de la (6R)-4,5,6,7-tétrahydro-3.6-diméthyl-3H-benzo[b]furan-2-one, c'est à dire de la lactone (I), des documents de l'art antérieur utilisent cette dénomination pour désigner la

5,6,7,7a-tétrahydro-7a-hydroxy-3,6-diméthyl-4H-benzo[b]furan-2-one de formule (II).

Ainsi par exemple Ito et al, dans Kitami Kogyo Tanki Daigaku. Kenkyu Hokoku, (1969), 2(4), 585-587 qui décrivent "l'isolation de la menthofurolactone à partir de la *Mentha Arvensis*" associent la structure (II) à la dénomination "menthofurolactone" et décrivent également sa synthèse par oxydation du menthofurane. La confusion vient sans doute du fait que, comme il est mentionné plus haut, tant la lactone (I) que l'hydroxylactone (II) sont des produits de l'oxydation du menthofurane et, selon l'art antérieur, la lactone (I) s'oxyde très facilement en l'hydroxylactone (II). En conséquence, les documents de l'art antérieur de C. Shu et al. et K. Umemoto, qui citent la menthofurolactone comme constituant de produit naturel sans en décrire la structure et qui sont postérieurs au document précédemment cité de Ito et al. ne permettent pas d'établir quelle était la structure de la lactone détectée et désignée par "menthofurolactone". Or, il apparaît d'après les conditions expérimentales utilisées dans l'art antérieur que le composé détecté dans les produits naturels se trouve être l'hydroxylactone de formule (II). Cependant, nous avons découvert de façon tout à fait surprenante que la lactone de formule (I) est présente dans la *Menthe poivrée concrete* à l'état de traces, bien que ce composant soit très difficile à détecter. En particulier, nous avons pu noter que seules certaines conditions expérimentales en ce qui concerne le choix de la colonne capillaire GC, ont permis de détecter le produit en chromatographie gazeuse couplée à la spectrométrie de masse GC-MS. Ainsi, lors d'une analyse GC-MS effectuée au moyen d'une colonne polaire telle que celles utilisées dans les documents de l'art antérieur qui divulguent des analyses de produits naturels, la lactone (I) n'est pas détectée. En revanche, la même analyse GC-MS menée sur colonne apolaire nous a permis d'observer la présence de la (6R)-4,5,6,7-tétrahydro-3,6-diméthyl-3H-benzo[b]furan-2-one. A l'issue de cette comparaison, il apparaît clairement que les deux documents cités plus haut désignent par "menthofurolactone" l'hydroxylactone (II). Le brevet EP 0 041 122 décrit une composition de parfum, caractérisée par une teneur de 1 à 50% en poids en dérivés de di- et tétrahydrobenzofuranone.

Ainsi, bien que de structure connue, aucun document de l'art antérieur ne décrit une synthèse optimale de la lactone (I) qui éviterait son oxydation rapide en hydroxylactone (II); par ailleurs il semble que cette lactone n'ait jamais été identifiée comme constituant d'un produit naturel. Enfin, il n'existe aucune mention ou suggestion dans l'art antérieur quant à l'utilisation de la (6R)-4,5,6,7-tétrahydro-3,6-diméthyl-3H-benzo[b]furan-2-one à titre d'ingrédient parfumant ou aromatisant.

## Exposé de l'invention

Or, nous avons maintenant découvert de façon tout à fait surprenante que la lactone de formule (I) possède d'excellentes propriétés organoleptiques qui rendent son utilisation très intéressante dans la parfumerie ainsi que dans l'industrie des arômes.

La présente invention a ainsi pour objet l'utilisation de la (6R)-4,5,6,7-tétrahydro-3,6-diméthyl-3H-benzo[b]furan-2-one à titre d'ingrédient parfumant ou aromatisant.

La (6R)-4,5,6,7-tétrahydro-3,6-diméthyl-3H-benzo[b]furan-2-one a une odeur menthée. légèrement vanillée dont le goût apporte douceur et arrondi aux compositions auxquelles elle est ajoutée et permet d'obtenir des effets organoleptiques très prononcés et typiques lorsque ledit composé est incorporé dans un parfum ou un arôme, et cela même à haute dilution.

Plus précisément, la lactone (I) développe une odeur phénolique-coumarinée, qui la rend utile pour un emploi alternatif à celui de la coumarine. La (6R)-4,5,6,7-tétrahydro-3,6-diméthyl-3H-benzo[b]furan-2-one possède en outre une note odorante de type menthé tout à fait originale qui la rend très appréciée des parfumeurs. En effet. cette combinaison de caractères odorants permet d'impartir différentes nuances olfactives dont on n'aurait pu disposer, en principe, qu'avec la contribution de plusieurs composés dont la combinaison produirait cependant des effets olfactifs inévitablement différents.

L'odeur du composé de l'invention est par ailleurs avantageusement puissante.

La lactone de formule (I) se prête aussi bien à une utilisation en parfumerie fine, dans les parfums, eaux de toilette ou lotions après-rasage, qu'à d'autres emplois courants en parfumerie tels que le parfumage des savons, gels de douche ou de bain, de produits d'hygiène, ou de traitement des cheveux comme les shampoings ou après-shampoings, ainsi que de déodorants corporels et déodorants ambiants, ou encore de préparations cosmétiques.

La (6R)-4,5,6,7-tétrahydro-3,6-diméthyl-3H-benzo[b]furan-2-one peut également être employée dans les applications telles que les détergents liquides ou solides destinés au traitement de textiles, adoucissants textiles, ou encore dans des compositions détergentes ou produits d'entretien destinés au nettoyage de la vaisselle ou de surfaces variées, à usages domestiques ou industriels.

Dans ces applications, le composé de l'invention peut être utilisé seul ou en mélange avec d'autres ingrédients parfumants, des solvants ou adjuvants d'usage courant en parfumerie. La nature et la variété de ces coingrédients n'appellent pas ici une description plus détaillée, qui ne saurait d'ailleurs être exhaustive, l'homme du métier étant à même de les choisir de par ses connaissances générales et en fonction de la nature du produit à parfumer et de l'effet olfactif désiré. Ces coingrédients parfumants peuvent appartenir à des classes chimiques aussi variées que les alcools,

aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont répertoriés dans des textes de référence tels que le livre de S. Arctander, Perfume and Flavor Chemicals, 1969, Montclair, New Jersey, USA, ou ses versions plus récentes, ou dans d'autres oeuvres de nature similaire.

Les proportions dans lesquelles le composé selon l'invention peut être incorporé dans les produits divers susmentionnés varient dans une gamme de valeurs très étendue. Ces valeurs dépendent de la nature de l'article ou produit que l'on veut parfumer et de l'effet olfactif recherché, ainsi que de la nature des coingrédients dans une composition donnée, lorsque le composé de l'invention est utilisé en mélange avec des coingrédients parfumants, des solvants ou des adjuvants d'usage courant dans l'art.

A titre d'exemple, on peut citer des concentrations typiques de l'ordre de 1 à 10% en poids, voire même 20% en poids du composé de l'invention, par rapport au poids de composition parfumante dans laquelle il est incorporé. Des concentrations bien inférieures à celles-ci peuvent être utilisées lorsque ce composé est directement appliqué dans le parfumage des produits de consommation divers cités auparavant.

Le composé de l'invention s'avère également très utile dans de domaine des arômes.

Son goût est typiquement associé avec les goûts caractéristiques de la vanille ou du chocolat. La lactone de l'invention apporte aux compositions auxquelles elle est ajoutée, une douceur et un arrondi très appréciés des aromaticiens.

Grâce à ses caractéristiques, la (6R)-4,5,6,7-tétrahydro-3,6-diméthyl-3H-benzo[b]furan-2-one se prête particulièrement bien aux applications dans le domaine des arômes où un goût sucré est souhaité, comme par exemple dans des desserts, bonbons et autres produits de confiserie, compotes, des yaourts et autres produits laitiers. des chewing-gums, des confitures. La lactone (I) se prête également à une utilisation dans les boissons, les produits glacés, les cigarettes, le tabac à chiquer, les préparations pharmaceutiques et les produits de soins dentaires.

Dans ces applications, le composé de l'invention est typiquement utilisé dans des concentrations de l'ordre de 1 ppb à 100 ppm, de préférence 10 ppb à 20 ppm par rapport aux aliments dans lesquels il est incorporé. Des concentrations bien supérieures à celles-ci peuvent être utilisées lorsque le composé de l'invention est utilisé dans les arômes ou compositions aromatisantes concentrés qui seront incorporés dans des produits de consommation.

Un autre objet de l'invention concerne un procédé original de fabrication de la (6R)-4,5,6,7-tétrahydro-3,6-diméthyl-3H-benzo[b]furan-2-one. Comme mentionné plus haut, l'art antérieur ne fournit aucune solution optimale au problème de la synthèse de la lactone (I), celle-ci étant susceptible de s'oxyder en l'hydroxylactone correspondante lorsqu'elle est préparée dans les conditions décrites dans l'art antérieur. La présente invention fournit une solution au problème cité et concerne ainsi un procédé de préparation de la (6R)-4,5,6,7-tétrahydro-3,6-diméthyl-3H-benzo[b]furan-2-one caractérisé en ce que l'on effectue une oxydation enzymatique du menthofurane. En particulier, l'utilisation d'une lipase de type *Novozym 435* dans des conditions réactionnelles qui seront décrites plus précisément dans l'Exemple 1 ci-après, permet d'obtenir la lactone (I) lors d'une réaction d'oxydation réalisée de manière contrôlée de façon à limiter la formation de l'hydroxylactone (II).

L'invention sera maintenant décrite de façon plus détaillée à l'aide des exemples suivants dans lesquels les températures sont indiquées en degrés Celsius et les abréviations ont le sens usuel dans l'art.

## Manières de réaliser l'invention

### Exemple 1

Préparation de la (6R)-4,5,6,7-tétrahydro-3,6-diméthyl-3H-benzo[b]furan-2-one

On a dilué 150 g (1 mole) de (R)-(+)-menthofurane (origine synthétique : Toyotama Perf., $[\alpha]^D_{20}$=84,1°; ou naturelle : Todd $[\alpha]^D_{20}$=88,1°), 144 g (1 mole) d'acide octanoique et 7 g (11500 U/g, 80500 unités) de lipase *Novozym 435* immobilisée, dans 150 ml de toluène. On a ensuite ajouté goutte à goutte au mélange réactionnel, 142 ml (1,46 mole) de péroxyde d'hydrogène à 35%, en 15 min, à 45°. On a assisté à une réaction exothermique. On a alors maintenu le mélange réactionnel à température ambiante durant 8 h. Après filtration, on a ajouté 600 ml de diéthyléther, puis la phase organique a été lavée successivement avec du $Na_2CO_3$ à 5%, de la saumure, de l'eau et à nouveau de la saumure. On a obtenu 148,3 g d'une huile jaune. Après distillation sous vide sur 1 g de 2,6-di-tert-butyl-4-méthylphenol (BHT), on a obtenu 37,2 g de (6R)-4,5,6,7-tétrahydro-3,6-diméthyl-3H-benzo[b]furan-2-one sous forme d'un mélange environ 1:1 de 2 diastéréoisomères, à 95% pure à partir de menthofurane synthétique, respectivement à 85% pure à partir de menthofurane naturel, avec un rendement de 22%.

Données analytiques :

$[\alpha]^D_{20}$ (c = 1, EtOH) = +70° (ex Todd) et +75° (ex Toyotama)
P.eb. : 48-50° (6 Pa)

RMN($^1H$, $CDCl_3$) : 3,08(m, 1H); 2,33(m, 1H); 2,07(m, 2H) ; 1.89(m, 2H) ; 1,79(m, 1H); 1,37(m, 1H) ; 1,30 et 1,29 (2d, J=7,9 et 7,5Hz, 3H, 2 diastéréoisomères dans un rapport 1/1) ; 1,06(d, J=6,3Hz, 3H).
RMN($^{13}C$, $CDCl_3$) : 180,6 et 180,5(2s, 2 diast.) ; 148,4(s); 115,3(s); 41,1 et 40,7(d) ; 30,6 et 30,4(t) ; 30,13 et 30,08 (t) ; 29,16 et 29,11(d) ; 21,3(q); 20,7 et 20,5(t); 14,2(q).
SM(EI) : 166(100), 151(5), 138(26), 123(39), 110(30), 95(60), 81(85), 67(26), 55(13).

Exemple 2

Préparation de pudding aromatisé à la vanille

On a préparé une composition de base pour pudding à partir des ingrédients suivants, en utilisant des techniques courantes :

| Ingrédients | % en poids |
|---|---|
| Lait entier | 81,26 |
| Crème à 35% de matière grasse | 4,50 |
| Lait en poudre écrémé | 1,80 |
| Fécule de maïs modifiée (Clearam CH 20) 1) | 2,00 |
| Sucre | 10,00 |
| Satiagum ADC 25 2) | 0,40 |
| Sel | 0,04 |
| Total | 100,00 |

1) origine : Roquette Frères, Lille, France
2) carrageenan ; origine : S.B.I., Boulogne Billancourt, France

On a ensuite ajouté 0.03% d'arôme de vanille de formule suivante :

| Ingrédients | % en poids |
|---|---|
| Acétylméthylcarbinol | 5 |
| Aldéhyde anisique à 10% * | 5 |
| Diacétyle | 3 |
| Vanilline | 50 |
| Extrait naturel de vanille | 500 |
| Alcool éthylique | 437 |
| Total | 1000 |

* dans l'éthanol

Cet arôme de vanille a été évalué dans la préparation pour pudding, puis comparé à l'aveugle par des experts aromaticiens, à un arôme identique auquel on a ajouté 0,005% de (6R)-4,5,6,7-tétrahydro-3,6-diméthyl-3H-benzo[b]furan-2-one, dosé de la même façon à 0,03% dans la préparation pour pudding.
De l'avis des aromaticiens, l'aromatisation contenant la (6R)-4,5,6,7-tétrahydro-3,6-diméthyl-3H-benzo[b]furan-2-one a une tonalité plus douce et plus arrondie que l'aromatisation témoin.

Exemple 3

Préparation de pudding aromatisé au chocolat

On a préparé une composition de base pour pudding à partir des ingrédients suivants, en utilisant des techniques courantes :

| Ingrédients | % en poids |
|---|---|
| Lait entier | 78,94 |
| Crème à 35% de matière grasse | 3,00 |

(suite)

| Ingrédients | % en poids |
|---|---|
| Lait en poudre écrémé | 1,70 |
| Fécule de maïs modifiée (Clearam CH 20) 1) | 1,70 |
| Sucre | 11,50 |
| Cacao de type Zaan D 11CM (10-12% de matière grasse) 2) | 1,80 |
| Chocolat noir | 1,00 |
| Satiagum ADC 25 3) | 0,32 |
| Sel | 0.04 |
| Total | 100,00 |

1) origine : Roquette Frères, Lille, France
2) origine : Cocoafabrik de Zaan B.V., Koogan de Zaan, Pays-Bas
3) carrageenan ; origine : Sanofi Bio-Ind., Grasse, France

On a ensuite ajouté 0,03% d'arôme chocolat de formule suivante :

| Ingrédients | % en poids |
|---|---|
| Extrait naturel de cacao | 560 |
| Extrait naturel de vanille | 100 |
| Extrait de caroube | 125 |
| Furanéol ® 1) à 10% * | 3 |
| Vanilline | 20 |
| Alcool éthylique | 192 |
| Total | 1000 |

* dans l'éthanol
1) 4-hydroxy-2,5-diméthyl-3(2H)-furanone ; origine : Firmenich SA, Genève, Suisse

Cet arôme chocolat a été évalué dans la préparation pour pudding, puis comparé à l'aveugle par des experts aromaticiens, à un arôme identique auquel on a ajouté 0,005% de (6R)-4,5,6,7-tétrahydro-3,6-diméthyl-3H-benzo[b]furan-2-one, toujours dosé à 0,03% dans la préparation pour pudding.
De l'avis des aromaticiens, l'aromatisation contenant la (6R)-4,5,6,7-tétrahydro-3,6-diméthyl-3H-benzo[b]furan-2-one a, de même que dans l'Exemple 2. une tonalité plus douce et plus arrondie que l'aromatisation témoin.

Exemple 4

Préparation de chewing-gum

On a préparé du chewing-gum à partir des ingrédients suivants :

| Ingrédients | % en poids |
|---|---|
| Base de type Cafosa Dorada Plus T205-01 1) | 18,00 |
| Sucre (taille de particule : 50 microns) | 61,50 |
| Glucose | 20,00 |
| Glycérine à 85% | 0,50 |
| Total | 100,00 |

Une essence de Menthe Arvensis a été évaluée à 1% dans cette base de chewing-gum. A cette essence de menthe, on a ajouté 0,06% de (6R)-4,5,6,7-tétrahydro-3,6-diméthyl-3H-benzo[b]furan-2-one et on l'a ajoutée à 1% dans la même base de chewing-gum. Une évaluation à l'aveugle d'un panel d'expert a montré que l'échantillon contenant la (6R)-4,5,6,7-tétrahydro-3,6-diméthyl-3H-benzo[b]furan-2-one présentait avantageusement une note plus herbale et plus douce rappelant la Menthe Poivrée.

1) origine : Cafosa Gum Products Technology, Barcelone, Espagne.

Exemple 5

Préparation d'une composition parfumante pour une eau de toilette

On a préparé une composition de base à partir des ingrédients suivants :

| Ingrédients | Parties en poids |
|---|---|
| Acétate de styrallyle | 80 |
| Aldéhyde C12 à 50% * | 20 |
| Aldéhyde hexylcinnamique | 190 |
| Aldéhyde MNA à 10% * | 80 |
| Allyl amyl glycolate à 10% * | 60 |
| Essence d'aspic | 50 |
| Citronellol | 370 |
| Cyclohexylpropionate d'allyle | 10 |
| Dihydromyrcenol | 300 |
| Géraniol | 120 |
| Géranyle nitrile | 20 |
| Habanolide ® 1) | 80 |
| Hédione ® 2) | 200 |
| Linalol | 400 |
| Nonalactone $\gamma$ à 10% * | 20 |
| Essence de petitgrain | 200 |
| Phénethylol | 350 |
| Essence d'orange | 120 |
| Salicylate de cis-3-héxenyle | 30 |
| Terpinéol | 50 |
| Triplal ® 3) à 10% * | 20 |
| Vert de lilas 4) | 30 |
| Total | 2800 |

* dans le dipropylène glycol

1) pentadécénolide ; origine : Firmenich SA, Genève, Suisse

2) dihydrojasmonate de méthyle ; origine : Firmenich SA, Genève, Suisse

3) 2,4-diméthyl-3-cyclohexèn-1-carboxaldéhyde ; origine: International Flavors & Fragrances, USA

4) (2,2-diméthoxyéthyl)benzène ; origine : Firmenich SA, Genève, Suisse

L'addition de 200 parties en poids de (6R)-4,5,6,7-tétrahydro-3,6-diméthyl-3H-benzo[b]furan-2-one à cette composition confère à son odeur une connotation poudrée-coumarinée accompagnée en outre d'une jolie fraîcheur herbacée.

Exemple 6

Préparation d'une composition parfumante

On a préparé une composition de base aux accords de type fleuri-héliotrope (tournesol), à partir des ingrédients suivants :

| Ingrédients | Parties en poids |
|---|---|
| Alcool cinnamique à 50% * | 120 |
| Essence de benjoin siam à 50% * | 100 |

* dans le dipropylène glycol

(suite)

| Ingrédients | Parties en poids |
|---|---|
| Géraniol | 100 |
| Héliotropine | 200 |
| Ionone β | 30 |
| Muscénone [1)] | 50 |
| Phénethylol | 150 |
| Terpinéol α | 60 |
| Essence d'ylang | 150 |
| Total | 960 |

1) 3-méthyl-cyclopentadécénone ; origine : Firmenich SA, Genève. Suisse

A 960 parties en poids de cette composition fleurie-héliotrope, on ajoute 40 parties en poids de (6R)-4,5,6,7-tétrahydro-3,6-diméthyl-3H-benzo[b]furan-2-one, ce qui confère à la composition une jolie note herbacée, coumarinée avec un effet de fraîcheur menthée. La note balsamique conférée par l'héliotropine se trouve renforcée, alors que la connotation crésylique de l'essence d'ylang est bien couverte par une note plus douce.

## Revendications

1. Utilisation de la (6R)-4,5,6,7-tétrahydro-3,6-diméthyl-3H-benzo[b]furan-2-one à titre d'ingrédient parfumant ou aromatisant.

2. Produit parfumé contenant à titre d'ingrédient parfumant la (6R)-4,5,6,7-tétrahydro-3,6-diméthyl-3H-benzo[b]furan-2-one.

3. Produit parfumé selon la revendication 2, sous forme d'un parfum ou d'une eau de toilette, d'un savon, d'un gel de douche ou de bain, d'un shampooing ou autre produit d'hygiène capillaire, d'une préparation cosmétique, d'un déodorant corporel ou d'air ambiant, d'un détergent ou adoucissant textile ou d'un produit d'entretien.

4. Produit aromatisé contenant à titre d'ingrédient actif la (6R)-4,5,6,7-tétrahydro-3,6-diméthyl-3H-benzo[b]furan-2-one.

5. Produit aromatisé selon la revendication 4, sous forme d'un dessert, d'un bonbon ou autre produit de confiserie, d'une compote, d'un yaourt ou autre produit laitier, d'un chewing-gum, d'une confiture, d'une boisson, d'une glace, de cigarettes, de tabac à chiquer, d'une préparation pharmaceutique, d'un produit de soin dentaire.

6. Procédé pour conférer, améliorer ou modifier la rondeur et/ou la douceur d'une composition aromatisante ou d'un produit aromatisé, **caractérisé en ce qu'**on ajoute à ladite composition ou audit produit la (6R)-4,5,6,7-tétrahydro-3,6-diméthyl-3H-benzo[b]furan-2-one.

7. Procédé de préparation de la (6R)-4,5,6,7-tétrahydro-3,6-diméthyl-3H-benzo[b]furan-2-one **caractérisé en ce qu'**on effectue une oxydation enzymatique du (R)-menthofurane.

8. Procédé selon la revendication 7, **caractérisé en ce que** l'oxydation enzymatique est effectuée en présence d'une lipase de type Novozym 435.

## Claims

1. Use of (6R)-4,5,6,7-tetrahydro-3,6-dimethyl-3H-benzo[b]furan-2-one as perfuming or flavouring ingredient.

2. A perfumed article containing (6R)-4,5,6,7-tetrahydro-3,6-dimethyl-3H-benzo[b]furan-2-one as perfuming ingredient.

3. Perfumed article according to claim 2, in the form of a perfume or cologne, a soap, a bath or shower gel, a shampoo or other haircare article, a cosmetic preparation, a body or air deodorant, a detergent, a fabric softener or a household article.

4. A flavoured article containing (6R)-4,5,6,7-tetrahydro-3,6-dimethyl-3H-benzo[b]furan-2-one as active ingredient.

5. Flavoured article according to claim 4, in the form of a dessert, a candy or other confectionery product, a compote, a yoghurt or other dairy article, a chewing-gum, a jam, a drink, an ice-cream, cigarettes, a chewing tobacco, a pharmaceutical preparation, a dental care article.

6. Process for imparting, improving or modifying the roundness and/or sweetness of a flavouring composition or flavoured article, **characterised in that** (6R)-4,5,6,7-tetrahydro-3,6-dimethyl-3H-benzo[b]furan-2-one is added to said composition or article.

7. Process of preparation of (6R)-4,5,6,7-tetrahydro-3,6-dimethyl-3H-benzo[b]furan-2-one **characterised in that** an enzymatic oxidation of (R)-menthofurane is carried out.

8. Process according to claim 7, **characterised in that** the enzymatic oxidation is carried out in the presence of a lipase of the Novozym 435 type.

**Patentansprüche**

1. Verwendung von (6R)-4,5,6,7-Tetrahydro-3,6-dimethyl-3Hbenzo[b]furan-2-on als Duft- oder Aromabestandteil.

2. Parfümiertes Produkt, enthaltend als Duftbestandteil (6R)-4,5,6,7-Tetrahydro-3,6-dimethyl-3H-benzo[b]furan-2-on.

3. Parfümiertes Produkt nach Anspruch 2 in Form eines Parfüms oder eines Eau de Toilette, einer Seife, eines Dusch- oder Badegels, eines Shampoos oder eines anderen Haarpflegeprodukts, eines Kosmetikpräparats, eines Körperdeodorants oder eines Raumlufterfrischers, eines Waschmittels oder Textilweichspülers oder eines Pflegeprodukts.

4. Aromatisiertes Produkt, enthaltend als Wirkstoff (6R)-4,5,6,7-Tetrahydro-3,6-dimethyl-3H-benzo[b]furan-2-on.

5. Aromatisiertes Produkt nach Anspruch 4 in Form einer Nachspeise, eines Bonbons oder eines anderen Süßwarenprodukts, eines Breis, eines Joghurts oder eines anderen Milchprodukts, eines Kaugummis, einer Konfitüre, eines Getränks, eines Speiseeises, von Zigaretten, von Kautabak, eines pharmazeutischen Präparats, eines Zahnpflegeprodukts.

6. Verfahren, um einer Aromazusammensetzung oder einem aromatisierten Produkt Abgerundetheit und/oder Süße zu verleihen, diese zu verbessern oder zu modifizieren, **dadurch gekennzeichnet, daß** man der Zusammensetzung oder dem Produkt (6R)-4,5,6,7-Tetrahydro-3,6-dimethyl-3H-benzo[b]furan-2-on zusetzt.

7. Verfahren zur Herstellung von (6R)-4,5,6,7-Tetrahydro-3,6-dimethyl-3H-benzo[b]furan-2-on, **dadurch gekennzeichnet, daß** man eine enzymatische Oxidation von (R)-Menthofuran durchführt.

8. Verfahren nach Anspruch 7, **dadurch gekennzeichnet, daß** die enzymatische Oxidation in Gegenwart einer Lipase vom Typ Novozym 435 durchgeführt wird.